# EUROPÄISCHE PATENTSCHRIFT

(11) **EP 3 979 954 B1**
(45) Veröffentlichungstag und Bekanntmachung des Hinweises auf die Patenterteilung: **12.11.2025**
(21) Anmeldenummer: 20734459.9
(22) Anmeldetag: 05.06.2020
(51) Int. Cl.: A61F 2/68, A61B 5/11, A61F 2/70, A61F 2/72, A61N 1/36

(54) **VERFAHREN ZUM BETREIBEN EINER ORTHOPÄDIETECHNISCHEN EINRICHTUNG UND ENTSPRECHENDE EINRICHTUNG**
METHOD FOR OPERATING AN ORTHOPEDIC DEVICE AND CORRESPONDING DEVICE
PROCÉDÉ POUR FAIRE FONCTIONNER UN DISPOSITIF ORTHOPÉDIQUE ET DISPOSITIF CORRESPONDANT

(30) Priorität: 05.06.2019 DE 102019115096
(43) Veröffentlichungstag der Anmeldung: 13.04.2022
(73) Patentinhaber: Otto Bock Healthcare Products GmbH, 1110 Wien (AT); Asklepios Klinik Alsbach GmbH, 64665 Alsbach-Haehnlein (DE)
(72) Erfinder: ZAJC, Johannes, 1180 Wien (AT); AUGSTEN, Andreas, 60437 Frankfurt (DE)
(74) Vertreter: Gramm, Lins & Partner Patent- und Rechtsanwälte PartGmbB
(86) Internationale Anmeldenummer: PCT/EP2020/065687
(87) Internationale Veröffentlichungsnummer: WO 2020/245398

(56) Entgegenhaltungen:
- CN-A- 106 821 680
- DE-A1- 102014 117 663
- JP-A- 2017 205 213
- JP-B2- 6 168 488
- US-A1- 2009 043 357
- US-A1- 2018 042 654
- MEYNS PIETER ET AL: "The how and why of arm swing during human walking", GAIT & POSTURE, ELSEVIER, AMSTERDAM, NL, vol. 38, no. 4, 13 March 2013 (2013-03-13), pages 555 - 562, XP028733473, ISSN: 0966-6362, DOI: 10.1016/J.GAITPOST.2013.02.006

## Beschreibung

Die Erfindung betrifft ein Verfahren zum Betreiben einer orthopädietechnischen Einrichtung, die ein erstes Körperteil eines Trägers unterstützt oder ersetzt und wenigstens einen steuerbaren Aktuator aufweist, wobei das Verfahren die folgenden Schritte aufweist:
a) Bestimmen eines zeitlichen Verlaufs wenigstens eines Parameters, der eine Aussage über einen Bewegungszustand des Trägers erlaubt, aus Messwerten wenigstens eines Sensors,
b) Erkennen des Bewegungszustandes des Trägers aus dem wenigstens einen bestimmten zeitlichen Verlauf und
c) Steuern des wenigstens einen steuerbaren Aktuators in Abhängigkeit des erkannten Bewegungszustandes des Trägers.

Orthopädietechnische Einrichtungen sind insbesondere Orthesen, die für Extremitäten, also Arme und/oder Beine, eines Trägers hergestellt werden. Eine orthopädietechnische Einrichtung wird dabei in der Regel an einer einzigen Extremität, also einem einzigen Bein oder einem einzigen Arm, des Trägers angeordnet.

Eine Orthese unterstützt das jeweilige Körperteil. Dies umfasst einerseits das Stützen und Schützen vor Überbeanspruchung, beispielsweise in einem post-operativen Heilungsprozess, indem beispielsweise ein Winkelbereich, in dem ein Gelenk eingesetzt werden soll, durch eine Orthese begrenzt ist. Zum Unterstützen im Rahmen der vorliegenden Erfindung gehört auch das Stützen durch Entlasten, das beispielsweise bei Sport-Orthesen oder auch bei sogenannten Exoskeletten, also tragbaren und bei Bedarf mit Aktuatoren ausgestattete mechanische Strukturen, die im medizinischen Bereich beispielsweise in der Rehabilitation oder als Alternative zum Rollstuhl eingesetzt werden, erreicht wird. Es muss nicht zwangsweise eine Einschränkung des Anwenders vorliegen. Es ist beispielsweise auch mögliche eine solche Orthese / ein solches Exoskelett zu verwenden, um die Belastung des Körpers bei physischen Tätigkeiten zu reduzierten, die Leistungsfähigkeit zu steigern und/oder das Risiko einer Verletzung zu reduzieren.

Es ist wichtig und von großem Vorteil, den Bewegungszustand des Trägers zu kennen, um den steuerbaren Aktuator entsprechend anzupassen. Erkennbare Bewegungszustände sind beispielsweise aufwärts und abwärts Treppensteigen, das Gehen auf einer Rampe, das Gehen und Laufen in unterschiedlichen Geschwindigkeiten, Stehen, Sitzen, das Übersteigen von Hindernissen, die sich im Weg befinden oder auch eine für eine Routinearbeit charakteristische Bewegung. All diese unterschiedlichen Bewegungszustände benötigen oftmals unterschiedliche Steuerungen des steuerbaren Aktuators.

Wichtig ist dabei, zwischen einem Bewegungszustand des Trägers einer orthopädietechnischen Einrichtung, der oben beschrieben ist, und dem Bewegungszustand oder der Bewegung des jeweiligen Körperteils zu unterscheiden. Während des Gehens in der Ebene beispielsweise ändert sich der Bewegungszustand des Trägers der orthopädietechnischen Einrichtung nicht, so lange das Gehen in der Ebene andauert. Die Bewegung des Körperteiles, beispielsweise eines Knies hingegen ändert sich mehrfach in jedem Schritt. Es durchläuft Standphasen und Schwungphasen mit unterschiedlichen Schlüsselereignissen, wie beispielsweise dem Fersenauftritt. Auch das Erkennen und Vorhersagen dieser Schlüsselereignisse ist für die Steuerung einer orthopädietechnischen Einrichtung wichtig und aus dem Stand der Technik seit langem bekannt. Im Rahmen der hier beschriebenen Erfindung geht es in erster Linie jedoch um das Erkennen des Bewegungszustandes des Trägers der orthopädietechnischen Einrichtung und die Frage, wie ein Wechsel des Bewegungszustandes erkannt und die Steuerung der orthopädietechnischen Einrichtung entsprechend angepasst werden kann.

Der Bewegungszustand eines Trägers dauert folglich in der Regel mehrere Schrittzyklen lang an, während sich der Bewegungszustand eines Körperteils auf einer deutlich kürzeren Zeitskala ändert. Diese Änderung kann mehrfach innerhalb eines einzigen Schrittzyklus erfolgen. Der wenigstens eine steuerbare Aktuator ist vorzugsweise vorgesehen und eingerichtet, den Bewegungszustand eines Körperteils, nämlich des ersten Körperteils, das durch die orthopädietechnische Einrichtung unterstützt oder ersetzt wird, zu verändern. Er ist in der Regel nicht eingerichtet, um den Bewegungszustand des Trägers der orthopädietechnischen Einrichtung zu verändern.

Aus der DE 10 2014 117 663 A1 ist beispielsweise ein Verfahren bekannt, bei dem einzelne Muskeln mittel Elektrostimulation angeregt werden. Dabei wird es als wichtig angesehen, dies im richtigen Moment zu erreichen, so dass der Bewegungszustand eines Körperteils des Trägers, nicht jedoch der Bewegungszustand des Trägers selbst bestimmt wird. Ähnliches wird in der CN 106 821 680 A beschrieben.

Bei einem steuerbaren Aktuator kann es sich beispielsweise um ein Dämpfungselement, beispielsweise einen hydraulischen Dämpfer, handeln. Bei hydraulischen Dämpfern sind insbesondere Ventile in einer Fluidverbindung vorhanden, die vorzugsweise stufenlos geöffnet oder geschlossen werden können. Dadurch wird der Querschnitt der Fluidverbindung vergrößert oder verkleinert, wodurch der einem Fluidfluss entgegengesetzte Widerstand und damit die Dämpfung, die von dem Dämpfungselement hervorgerufen wird, verringert oder erhöht werden kann.

Bei dem steuerbaren Aktuator kann es sich auch um ein Stellglied handeln, durch das eine bestimmte Bewegung zumindest eines Teils der orthopädietechnischen Einrichtung oder der ganzen orthopädietechnischen Einrichtung gesteuert werden kann. Dies ist beispielsweise bei aktiven orthopädietechnischen Einrichtungen, beispielsweise aktiven Kniegelenken oder aktiven Knöchelgelenken nötig, damit das Gelenk der orthopädietechnischen Einrichtung die gewünschte Funktion erfüllt. Steuerbare Aktuatoren können aktiv oder passiv ausgebildet sein, unabhängig davon, ob es sich um Dämpfungselemente oder Stellglieder handelt.

Als steuerbare Aktuatoren im Sinne der vorliegenden Erfindung werden insbesondere auch Mittel und Methoden zur Stimulation des Bewegungsapparats angesehen, insbesondere der Elektrostimulation von Muskeln und Nerven, beispielsweise durch Elektroden. Diese können beispielsweise auf der Haut des Trägers angeordnet sein und durch elektrische Impulse unter der Haut liegende Muskeln stimulieren. Ebenso kann es sich um subkutane Elektroden handeln, beispielsweise am Nerv anliegende Elektroden.

Aus dem Stand der Technik ist seit langem bekannt, den steuerbaren Aktuator in Abhängigkeit des erkannten Bewegungszustandes zu steuern. Dazu ist der wenigstens eine Sensor eingerichtet, Messwerte aufzunehmen, aus denen wenigstens ein Parameter bestimmbar ist, dessen zeitlicher Verlauf Aussagen über einen Bewegungszustand erlaubt. Dabei muss der zeitliche Verlauf nicht über einen ganzen Schrittzyklus hinweg detektiert oder ausgewertet und dokumentiert werden. Der Parameter kann beispielsweise der Kniewinkel einer Knieorthese sein, der beispielsweise über einen Schrittzyklus hinweg gemessen und in der elektrischen Steuerung ausgewertet wird. Der dabei maximal auftretende Kniewinkel unterscheidet sich je nach Bewegungszustand. Der maximale Flexionswinkel, der beispielsweise bei einem Knie auftritt, ist deutlich größer, wenn der Träger der orthopädietechnischen Einrichtung eine Treppe nach oben steigt, als wenn er in der Ebene geht. Daraus kann auf den Bewegungszustand geschlossen werden, wobei diese Information dann verwendet wird, um den steuerbaren Aktuator beispielsweise in der Schwungphase des Beins so zu steuern, dass der Arm die gewünschte Bewegung ausführt.

Die mit der orthopädietechnischen Einrichtung versehene und mit ihr versorgte Extremität wird als ipsilaterale Extremität bezeichnet. Eine nicht mit der orthopädietechnischen Einrichtung versehene Extremität wird hingegen als kontralaterale Extremität bezeichnet. Dabei kann die kontralaterale Extremität zu der ipsilateralen Extremität korrespondieren, wenn beide Extremitäten beispielsweise Arme sind. Ein nicht durch die orthopädietechnische Einrichtung versorgtes Bein wird jedoch auch dann als kontralateral bezeichnet, wenn die ipsilaterale Extremität ein Arm ist. Dies gilt im Sinne der vorliegenden Erfindung bevorzugt auch dann, wenn beide auf der gleichen Körperseite liegen, es sich also um einen linken Arm und ein linkes Bein oder einen rechten Arm und ein rechtes Bein handelt.

Herkömmlicherweise werden über den wenigstens einen Sensor Messwerte aufgenommen, aus denen Parameter der ipsilateralen Extremität bestimmt werden können. So kann beispielsweise der Kniewinkel, der Knöchelwinkel, verschiedene Momente, Relativpositionen unterschiedlicher Bauteile zueinander oder Geschwindigkeiten, Beschleunigungen oder Verlagerungen bestimmter Punkte der orthopädietechnischen Einrichtung relativ zueinander oder absolut bestimmt werden. All diese Parameter können verwendet werden, um den Bewegungszustand zu ermitteln. Nachteilig ist jedoch, dass der Bewegungszustand erst dann ermittelt werden kann, wenn die Messwerte erfasst wurden, also erst nach oder in dem jeweiligen Schritt. Die Bestimmung des Bewegungszustandes ist daher immer erst rückwirkend möglich. Eine Steuerung des steuerbaren Aktuators in Abhängigkeit des erkannten Bewegungszustandes basiert dabei immer auf der Voraussetzung, dass der Bewegungszustand des Trägers sich zwischen den beiden Schritten nicht ändert. Der in einem Schrittzyklus erkannte Bewegungszustand wird als auch für den nächsten Schrittzyklus gültig angesehen. Nachteile treten dann auf, wenn dies nicht der Fall ist und sich der Bewegungszustand ändert. Verfügt der Träger der orthopädietechnischen Einrichtung beispielsweise über wenigstens einen vollständig oder teilweise gelähmten Arm, kann dieser gegebenenfalls an der Schwingbewegung nicht teilnehmen. Dies hat zur Folge, dass Ungleichverteilungen bei Momenten und Beschleunigungen und gegebenenfalls ein Ungleichgewicht hervorgerufen werden. Diese werden vom Körper des Trägers ausgeglichen, wodurch es zu Fehlhaltungen und Schädigungen, Verspannungen und/oder Schmerzen, insbesondere im Hüftbereich, Schulterbereich und/oder im Bereich der Wirbelsäule kommen kann. Auch die hervorzurufende Illusion einer natürlichen Bewegung ist auf diese Weise nicht oder nur sehr schwer aufrechtzuerhalten.

Die JP 2017-205 213 A befasst sich mit einem Verfahren, das beispielsweise zum Verbessern eines Golfschwunges oder eines Laufstils verwendet werden kann. Die JP 6 168 488 B2 hingegen beschreibt ein Verfahren, mit dem beispielsweise die Balance des Benutzers des Verfahrens untersucht werden kann.

Der vorliegenden Erfindung liegt daher die Aufgabe zugrunde, ein Verfahren zum Betreiben einer orthopädietechnischen Einrichtung so weiterzuentwickeln, dass die Erkennung des Bewegungszustandes verbessert wird und die Bewegung, die der Träger der orthopädietechnischen Einrichtung mit der orthopädietechnischen Einrichtung ausführt, der natürlichen Bewegung möglichst gleicht.

Die Erfindung löst die gestellte Aufgabe durch ein Verfahren zum Betreiben einer orthopädietechnischen Einrichtung gemäß dem Oberbegriff des Anspruchs 1, die sich dadurch auszeichnet, dass zum Erkennen des Bewegungszustandes des Trägers zumindest auch der zeitliche Verlauf wenigstens eines Parameters eines zweiten Körperteiles des Trägers verwendet wird, wobei der wenigstens eine steuerbare Aktuator wenigstens eine Elektrode zur Elektrostimulation aufweist, die derart angesteuert wird, dass zumindest ein Arm des Trägers in Abhängigkeit des erkannten Bewegungszustandes des Trägers zum Schwingen gebracht wird.

Vorzugsweise weist der wenigstens eine steuerbare Aktuator wenigstens eine Elektrode zur Elektrostimulation auf, die derart angesteuert wird, dass ein Arm des Trägers in Abhängigkeit des erkannten Bewegungszustandes zum Schwingen gebracht wird. Dies ist insbesondere jedoch nicht ausschließlich für Träger interessant, die eine Lähmung des versorgten Armes, der die ipsilaterale Extremität ist, aufweisen.

Beim menschlichen Gang einer gesunden Person werden nicht nur die beiden Beine koordiniert bewegt. Gleichzeitig schwingen auch die beiden Arme nach vorn und hinten. Die Arme schwingen dabei zueinander versetzt, so dass ein Arm nach vorn schwingt, wenn der andere Arm nach hinten schwingt.

Die genannten Nachteile können dadurch verhindert werden, dass die wenigstens einen Elektrode zur Elektrostimulation, die den steuerbaren Aktuator bildet, so gesteuert wird, dass der versorgte Arm schwingt, wenn ein entsprechender Bewegungszustand, beispielsweise Gehen, erkannt wird. Dabei wird vorzugsweise der Deltoideusposterioris für ein Schwingen nach hinten und der Deltoideus-anterioris für ein Schwingen nach vorn stimuliert.

Selbstverständlich können auch aktive Orthesen entsprechende gesteuert werden, und so zu einem physiologischen Gang und einem natürlichen Gangbild beitragen.

Erfindungsgemäß wird der zeitliche Verlauf eines Parameters zum Erkennen des Bewegungszustandes verwendet. Eine Einzelmessung, die einen Messwert zu einem einzelnen Zeitpunkt liefert, ist nicht ausreichend. Es ist von Vorteil, jedoch nicht notwendig, wenn der zeitliche Verlauf des Parameters insbesondere bei sich wiederholenden Bewegungen über einen oder besonders bevorzugt mehrere Zyklen, beispielsweise Schrittzyklen bestimmt wird. Dies geschieht in der Regel durch eine Mehrzahl, vorzugsweise eine Vielzahl von Einzelmessungen, die den Messwert zu jeweils einem einzelnen Zeitpunkt liefern. Diese Ergebnisse der Einzelmessungen werden gespeichert und als zeitlicher Verlauf ausgewertet. Die Mehrzahl von Einzelmessungen kann dabei zeitlich äquidistant erfolgen. Der Abstand zwischen zwei Einzelmessungen muss dabei klein gegenüber der Länge beispielsweise eines Schrittzyklus sein, sodass ein zeitlicher Verlauf des Parameters aus der Mehrzahl der Einzelmessungen ermittelt werden kann.

Oftmals ist es von Vorteil und ausreichend, den zeitlichen Verlauf des Parameters nicht über ganze Schrittzyklen, sondern beispielsweise nur über bestimmte Teile eines Schrittzyklus zu bestimmen. Zum Erkennen eines Bewegungszustandes ist es oftmals ausreichend, den Parameter zu ganz bestimmten Zeitpunkten beispielsweise eines Schrittzyklus zu kennen. Diese bestimmten Zeitpunkte können beispielsweise der Fersenauftritt auf dem Boden oder der Zeitpunkt des Ablösens der Zehen sein. Um diesen Zeitpunkt möglichst exakt zu ermitteln ist es notwendig oder mindestens vorteilhaft, den zeitlichen Verlauf des Parameters in einem bestimmten Zeitraum vor und nach diesem bestimmten Zeitpunkt zu messen und zu bestimmen. Auch dies fällt unter die erfindungsgemäße Definition eines zeitlichen Verlaufes.

Ist der Bewegungszustand des Trägers, also insbesondere die Art seines Fortbewegens, erkannt worden, kann der steuerbare Aktuator entsprechend gesteuert werden. Dabei werden bevorzugt nicht einfache Routinen und zeitliche Abläufe, die für bestimmte Bewegungszustände in einem Datenspeicher einer elektronischen Steuereinheit hinterlegt sind, ausgeführt. Vielmehr wird bevorzugt der zeitliche Verlauf des Parameters des zweiten Körperteiles zur Steuerung des wenigstens einen steuerbaren Aktuators verwendet. Auf diese Weise wird erreicht, dass der durch die orthopädietechnische Einrichtung unterstützte oder ersetzte Körperteil harmonisch, natürlich und möglichst optimal an die Bewegungen der anderen Körperteile, insbesondere des zweiten Körperteils, angepasst bewegt wird. So wird beispielsweise ein natürliches Gangbild erzeugt, indem beispielsweise die Bewegung einer orthopädietechnischen Einrichtung an die Bewegung eines gesunden Beines, das in diesem Fall das zweite Körperteil bildet, angepasst wird. Alternativ oder zusätzlich dazu kann das zweite Körperteil auch ein Arm sein, dessen natürliche Schwenkbewegung beim Gehen oder Laufen verwendet wird, um die Bewegung einer Armorthese zu steuern.

Mit dem erfindungsgemäßen Verfahren ist es in besonders bevorzugten Ausgestaltungen folglich möglich, nicht nur den Bewegungszustand des Trägers möglichst frühzeitig zu erkennen und den wenigstens einen steuerbaren Aktuator entsprechend zu steuern, sondern die Bewegung des Aktuators an die Bewegung unterschiedlicher Körperteile anzupassen und so die Akzeptanz der orthopädietechnischen Einrichtung beim Träger zu vergrößern.

In einer bevorzugten Ausgestaltung ist der wenigstens eine Parameter eine Relativposition, Relativbewegung und/oder Relativgeschwindigkeit und/oder Relativbeschleunigung und/oder ein Relativwinkel des zweiten Körperteiles zu dem ersten Körperteil und/oder eines ersten Teils des zweiten Körperteiles zu einem zweiten Teil des zweiten Körperteils. Vorzugsweise handelt es sich bei dem zweiten Körperteil um einen Fuß, ein Knie, einen Oberschenkel, einen Unterschenkel und/oder eine Beinsehne. Das zweite Körperteil ist vorzugsweise eine unversorgte Extremität oder in Teil davon. Es kann jedoch auch von Vorteil sein, wenn das zweite Körperteil beispielsweise ein Teil einer Extremität ist, an der die orthopädietechnische Einrichtung angeordnet ist.

Um den zeitlichen Verlauf der Relativbewegung zu bestimmen, werden beispielsweise die Position der Körperteile zueinander und/oder ihre Lage in einem gemeinsamen Koordinatensystem relativ zu mindestens einem Sensor zu mehreren Zeitpunkten ermittelt. Unter Position wird insbesondere auch die translatorische und/oder rotatorische Orientierung zueinander verstanden werden.

Dabei ist es unerheblich, ob das zweite Körperteil, insbesondere die kontralaterale Extremität ebenfalls durch eine weitere orthopädietechnische Einrichtung versorgt ist.

Besonders interessant ist die Beinsehne eines unversorgten Beines als zweites Körperteil, die die gedachte Verbindungslinie zwischen einem Fuß und einer Hüfte der Extremität darstellt. Interessante Messgrößen der Beinsehne sind dabei insbesondere ihre Orientierung sowie ihre Länge sowie deren Geschwindigkeiten und Veränderungen. Die Beinsehne gibt einerseits Informationen über die Position des Fußes der kontralateralen Extremität in Relation zum Körperzentrum und zum Schwerpunkt. Sie gibt damit direkt und indirekt Informationen über die Progression und die Stabilität und/oder die Fußpositionierung des Trägers. Zudem ist die Bewegung der Beinsehne mit herkömmlichen Sensoren bereits zugänglich, auch wenn sie in der Regel nicht verwendet wird. Die Bewegung des proximalen Endpunktes der Beinsehne, also der Hüfte, kann bereits über vorhandene Sensorik, die in eine hier beschriebene orthopädietechnische Einrichtung integriert sein kann, berechnet werden. Über die Bewegung des distalen Endpunktes, also des Fußes der unversorgten Extremität, lassen sich insbesondere in der Standphase gute Annahmen treffen. **In** der Schwungphase kann die Bewegung, also insbesondere die Position und/oder die Änderung der Position, des Fußes durch den wenigstens einen Sensor bestimmt werden.

Sind der proximale Endpunkt und der distale Endpunkt der Beinsehne des unversorgten Beines bekannt, kann beispielsweise unter Zuhilfenahme bekannter Abmessungen des Oberschenkels und des Unterschenkels des Trägers der orthopädietechnischen Einrichtung auch ein Beinwinkel oder Kniewinkel bestimmt werden, der sich intuitiv interpretieren lässt und in Steuergesetzen verwendbar ist. Der Kniewinkel der versorgten Seite ist ein bewährter Steuerparameter.

Auch die relativen Positionen anderer Punkte relativ zueinander, beispielsweise die ipsilaterale Knieachse zum kontralateralen Fuß, kann von Interesse sein. Je mehr Sensoren verwendet werden, desto mehr unterschiedliche Parameter sind einer Messung zugänglich. Über kinematische Ketten kann auf andere Relativpositionen zurückgeschlossen werden, so dass weitere Parameter zugänglich werden.

Insbesondere aus den Relativpositionen und/oder Relativbewegungen sowie die Relativwinkeln in verschiedenen Kombinationen kann auch auf Segmentwinkel der kontralateralen Seite zurückgeschlossen werden. Dies betrifft beispielsweise den Oberschenkel, den Unterschenkel oder den Fuß. Daraus können Gelenkswinkel, beispielsweise der kontralaterale Hüftwinkel, der Kniewinkel oder der Knöchelwinkel bestimmt werden.

Durch die geschickte Wahl unterschiedlicher Sensoren zur Bestimmung unterschiedlicher Größen, aus denen die verschiedenen Parameter auch des zweiten Körperteiles bestimmt werden können, kann beispielsweise auf die kontralaterale Beinbewegung geschlossen werden.

Bevorzugt ist der wenigstens eine Sensor eingerichtet, einen Absolutwinkel, einen Relativwinkel, eine Geschwindigkeit, eine Beschleunigung, eine Kraft, einen Druck, eine Druckwelle, ein Moment, ein elektrisches Feld und/oder ein magnetisches Feld zu detektieren. Unter einer Druckwelle wird insbesondere auch eine Schallwelle, insbesondere eine Ultraschallwelle verstanden.

Es hat sich als vorteilhaft herausgestellt, wenn zum Bestimmen des wenigstens einen Parameters, insbesondere zum Bestimmen des wenigstens einen Parameters des zweiten Körperteils, bevorzugt der kontralateralen Extremität Daten der orthopädietechnischen Einrichtung und/oder des Trägers verwendet werden. Dies können beispielsweise Entfernungen, mögliche Schwenkwinkel oder Längenangaben sein. So ist es beispielsweise zur Bestimmung eines Kniewinkels eines kontralateralen Beins aus der Beinsehne notwendig, zumindest grob, vorzugsweise jedoch genau, die Unterschenkellänge und die Oberschenkellänge des Trägers der orthopädietechnischen Einrichtung auf der kontralateralen Seite zu kennen. Relative Größen der kontralateralen Seite zur ipsilateralen Seite können durch Messung von absoluten Messgrößen der ipsilateralen Seite ebenfalls in absolute Größen umgerechnet werden.

Vorzugsweise wird beim Betreiben der orthopädietechnischen Einrichtung wenigstens eine Steuergröße des wenigstens einen steuerbaren Aktuators auf einen Sollwert oder einen Sollwertverlauf gesteuert. Dieser ist vorteilhafterweise nicht nur vom erkannten Bewegungszustand selbst, sondern auch von dieser Erkennung zugrunde liegenden Parametern insbesondere von dem wenigstens einen Parameter des zweiten Körperteils, bevorzugt der kontralateralen Extremität abhängig.

Die Erfindung löst die gestellte Aufgabe zudem durch eine orthopädietechnische Einrichtung, die ein erstes Körperteil unterstützt oder ersetzt, wobei die orthopädietechnische Einrichtung wenigstens einen Sensor und eine elektrische Steuerung aufweist, die eingerichtet ist zum Durchführen eines hier beschriebenen Verfahrens.

Bei der Bestimmung des wenigstens einen Parameters der kontralateralen Extremität kann, wie bereits dargelegt, auf Berechnungen des entsprechenden Parameters aus Sensordaten zurückgegriffen werden. Alternativ oder zusätzlich dazu können fehlende Parameter, die mit den verwendeten Sensoren nicht direkt zugänglich sind, aus vorhandenen Messgrößen und gegebenenfalls einem Model oder Modelannahmen bestimmt werden. Die vorhandenen Messgrößen können dabei sowohl Messgrößen der ipsilateralen als auch der kontralateralen Seite sein. Entsprechende Modelle sind beispielsweise mechanische und kinematische Modelle, die die jeweiligen Bewegungen der Extremität beschreiben.

**In** einem Ausführungsbeispiel wird die Schwungbewegung eines Beins erkannt. Manche Hilfsmittel verfügen über keinerlei Kraftsensorik um festzustellen, ob sich die versorgte Seite in Bodenkontakt befindet. Die kontralaterale Beinbewegung kann Aufschluss darüber geben, ob es sich um ein nach hinten Gehen handelt, bei der das kontralaterale Standbein nach hinten abrollt, sodass die Stimulation des versorgten Armes entsprechend angepasst werden kann.

Die Erfindung befasst sich mit der Stimulation der Oberen Extremität - insbesondere, aber nicht beschränkt auf den Deltoideus - zur Unterstützung des Gangbildes. Dabei werden die Stimulationsamplitude, der zeitliche Verlauf der Stimulationsamplitude und/oder die Position der Stimulation in Abhängigkeit von Sensorwerten oder deren zeitlichen Verlauf bestimmt, wobei diese Sensorwerte von der aktuellen Gangphase abhängen. Als Sensoren kommen dabei insbesondere Inertial-Measurement-Units (IMU) in Frage. Durch die Positionierung am Ober- oder Unterschenkel kann zum Beispiel die relative Lage der Gliedmaße zum Boden bestimmt werden. Bei Verwendung von zumindest zwei IMUs kann zudem die relative Lage zweier Gliedmaßen zueinander bestimmt werden (z.B.: Kniewinkel, Fußgelenkswinkel). Dazu kann unter Umständen eine vorhergehende Kalibration der Sensoren notwendig sein.

Die Sensorwerte der in IMUs verbauten Beschleunigungssensoren und/oder Gyroskope können zudem verwendet werden um z.B.: die Momentangeschwindigkeit oder Winkelgeschwindigkeiten zu bestimmen und in Abhängigkeit davon die Stimulationsparameter festzulegen (z.B.: Amplitude, Position, Pulsform).

Mittels IMUs kann zudem der Zeitpunkt des Fersenauftritts bestimmt werden. Zur Bestimmung des Fersenauftritts hat sich auch die Verwendung eines sogenannten Fersenschalters bewährt - insbesondere im Zusammenhang mit Fallfußstimulatoren. In zeitlicher Anhängigkeit vom Fersenauftritt können patientenspezifische Stimulationsverläufe getriggert werden.

Die Sensorinformation wird in einem Rechner (z.B.: PC, Laptop, Microkontroller,...) verarbeitet und in entsprechende Ansteuerungssignale für den Stimulator umgewandelt. Dieser Rechner sollte im Idealfall portabel und kabellos (z.B.: via Bluetooth) mit den Sensoren verbunden sein.

Alternativ dazu könnte der Rechner direkt im Stimulator integriert sein.

Um die die Stimulationspunkte flexibel festzulegen können mehrere Stimulationselektroden an Schulter und Arm angebracht werden. Um einen einfachen Anlegeprozess zu gewährleisten können die Elektroden mittels Klettverschlüssen an Orthesen fixiert werden.

Bevorzugterweise werden jedoch sogenannte Elektroden Arrays verwendet, die mittels Multiplexer eine Positionierung sogenannter "Virtueller Elektroden" auch zwischen den physikalischen Elektroden des Arrays und somit eine fließende Verschiebung von Stimulationspunkten ermöglichen.

Die Erfindung wirkt durch Stimulation Subluxation und Spastiken entgegen. Insbesondere während des Gehens ermöglicht die Erfindung auch bei wenigstens einem schlaffen Arm ein physiologisches Mitschwingen des Armes, was das Gangbild verbessert und Folgeschäden entgegenwirkt.

**In** Abhängigkeit der tatsächlich verwendeten Sensoren und der damit erzielten Aussage über den momentanen Bewegungsstatus, kann die Erfindung auch in anderen Situationen unterstützend wirken. So kann etwa bei Drehungen, Stehenbleiben oder ähnlichen Situationen das gezielte Einbringen und/oder Nicht-Einbringen von Stimulation unter Anderem zu einem verbesserten Gleichgewicht beitragen.

Mit Hilfe der beiliegenden Zeichnungen werden nachfolgend einige Ausführungsbeispiele näher erläutert. Es zeigen:
- Figur 1 -: eine schematische Darstellung einer Anwendung eines hier beschriebenen Verfahrens,
- Figur 2 -: ein weiteres Beispiel einer Anwendung, sowie
- Figur 3 -: ein Flussdiagramm.

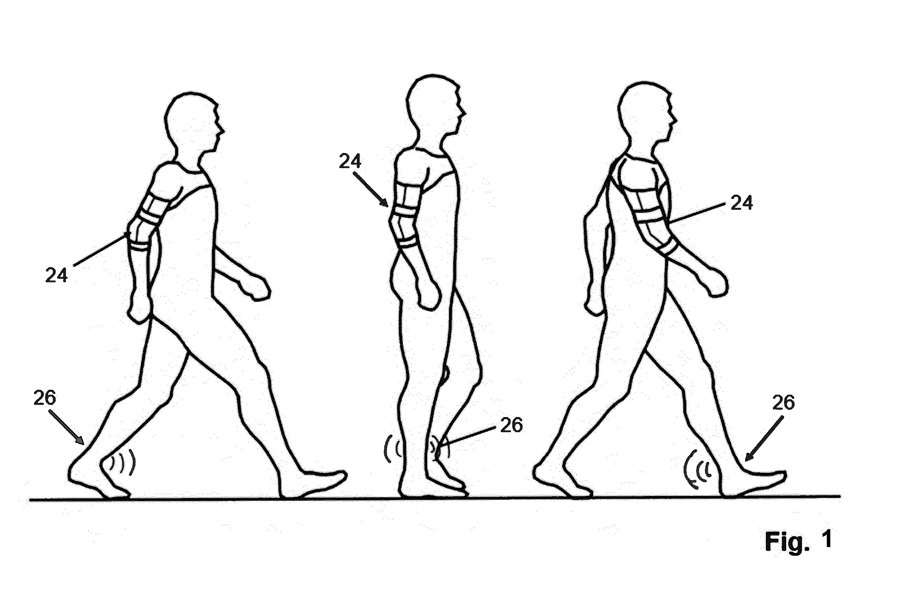

Figur 1 ist ein Beispiel dafür, dass der erste Körperteil 24, der im gezeigten Ausführungsbeispiel der rechte Arm des Trägers ist, nicht zwangsläufig "gegenüber" des zweiten Körperteils 26 liegen muss, der im gezeigten Ausführungsbeispiel der linken Knöchel ist. Figur 1 zeigt drei Positionen innerhalb eines Schrittzyklus, bei denen jeweils die Position des zweiten Körperteils 26, also des linken Knöchels, relativ zu einem weiteren Körperteil, nämlich dem rechten Knöchel, bestimmt wird. In der linken Darstellung der Figur 1 befindet sich der zweite Körperteil 26 hinter dem Rumpf des Trägers. Gleiches gilt für den ersten Körperteil 24. Die Relativposition des zweiten Körperteils 26 relativ zum rechten Knöchel wird bestimmt, was durch die drei kleinen Linien angedeutet wird. Im Verlauf eines Schrittzyklus verändert sich die Position des zweiten Körperteils 26 relativ zum rechten Knöchel über die in der Mitte der Figur 1 gezeigten Positionen innerhalb der Schwungphase bis zur in Figur 1 rechts dargestellte Position beim Fersenauftritt. Entsprechend wird auch die Bewegung des ersten Körperteils 24, das von einer Arm-Orthese unterstützt wird, gesteuert.

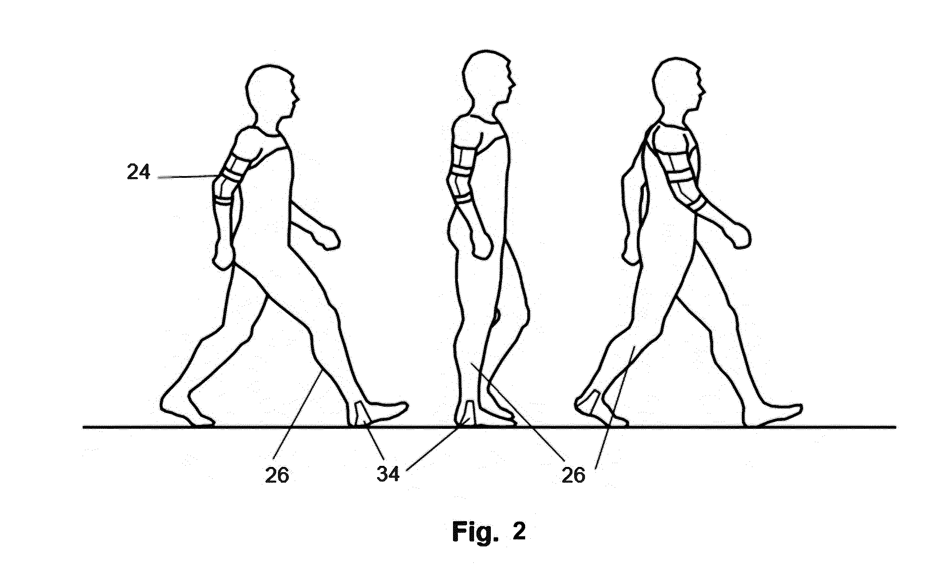

Figur 2 ist ein Beispiel dafür, dass sich das zweite Körperteil 26, auf dem ein Sensor 34 zur Bestimmung des Bewegungszustandes, insbesondere der Stand-Phase im Schrittzyklus, angebracht ist, auf der selben Körperhälfte befinden kann wie das Körperteil 24, das mit einem orthopädietechnischen Hilfsmittel ausgestattet ist. Dieser kann - wie beispielsweise im Falle eines Heel-Switches oder eines Inertialsensors - ausschließlich aufgrund von Messungen der mit dem Sensor 34 ausgestatteten Extremität 26 Informationen über den Bewegungszustand erfassen. Ebenso können mittels des am Körperteil 26 angebrachten Sensors 34 auch Messstrahlen empfangen werden, die vom gegenüberliegenden Bein ausgesandt oder reflektiert oder reemittiert werden.

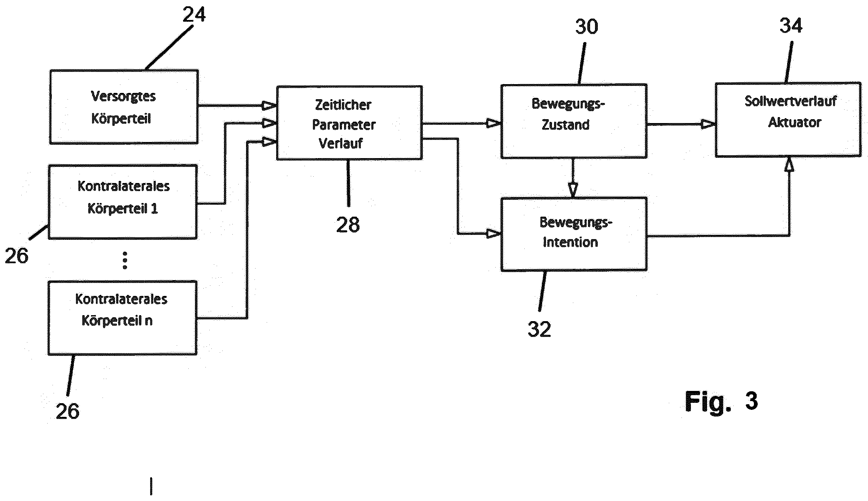

Figur 3 zeigt ein schematisches Flussdiagramm für ein hier beschriebenes Verfahren. Von einem ersten Körperteil 24 und mindestens einem zweiten Körperteil 26 werden Parameter ermittelt, deren zeitlicher Verlauf 28 bestimmt wird. Aus diesem wird sowohl ein Bewegungszustand 30 des Trägers als auch eine Bewegungsintention 32 bestimmt, wobei zur Bestimmung der Bewegungsintention 32 auch der bestimmte Bewegungszustand 30 herangezogen werden kann. Sowohl die Bewegungsintention 32 als auch der bestimmte Bewegungszustand 30 können getrennt voneinander oder in Kombination verwendet werden, um die Aktuator Steuerung 34 zu initiieren.

### Bezugszeichenliste

- 2: kontralaterale Extremität
- 4: ipsilaterale Extremität
- 6: Oberschenkelschaft
- 8: Kniegelenk
- 10: Unterschenkel
- 12: Fuß
- 14: Messstrahlung
- 16: Unterschenkelschaft
- 18: Oberschenkelrahmen
- 20: Unterschenkelrahmen
- 22: Kniegelenk
- 24: erster Körperteil
- 26: zweiter Körperteil
- 28: zeitlicher Verlauf
- 30: Bewegungszustand
- 32: Bewegungsintention
- 34: Sensor

## Patentansprüche

1. Verfahren zum Betreiben einer orthopädietechnischen Einrichtung, die
- ein erstes Körperteil (24) eines Trägers unterstützt und
- wenigstens einen steuerbaren Aktuator aufweist,
wobei das Verfahren die folgenden Schritte aufweist:
a) Bestimmen eines zeitlichen Verlaufes (28) wenigstens eines Parameters, der eine Aussage über einen Bewegungszustand (30) des Trägers erlaubt, aus Messwerten wenigstens eines Sensors (34),
b) Erkennen des Bewegungszustandes (30) des Trägers aus dem wenigstens einen bestimmten zeitlichen Verlauf (28) und
c) Steuern des wenigstens einen steuerbaren Aktuators in Abhängigkeit des erkannten Bewegungszustandes (30) des Trägers,
wobei zum Erkennen des Bewegungszustandes (30) des Trägers zumindest auch der zeitliche Verlauf (28) wenigstens eines Parameters eines zweiten Körperteiles (26) des Trägers verwendet wird, wobei der wenigstens eine steuerbare Aktuator wenigstens eine Elektrode zur Elektrostimulation aufweist, die derart angesteuert wird, dass zumindest ein Arm des Trägers in Abhängigkeit des erkannten Bewegungszustandes (30) des Trägers zum Schwingen gebracht wird, wobei der Bewegungszustand (30) des Trägers mehrere Schrittzyklen lang andauert.

2. Verfahren nach Anspruch 1 , **dadurch gekennzeichnet, dass** der wenigstens eine Parameter eine Relativposition, Relativbewegung und/oder Relativgeschwindigkeit und/oder Relativbeschleunigung und/oder ein Relativwinkel des zweiten Körperteiles (26) zu dem ersten Körperteil (24) und/oder eines ersten Teils des zweiten Körperteiles (26) zu einem zweiten Teil des zweiten Körperteils (26) ist.

3. Verfahren nach einem der vorstehenden Ansprüche, **dadurch gekennzeichnet, dass** der wenigstens eine Sensor (34) eingerichtet ist, einen Absolutwinkel, einen Relativwinkel, eine Geschwindigkeit, eine Beschleunigung, eine Kraft, einen Druck, eine Druckwelle, ein Moment, ein elektrisches Feld und/oder ein magnetisches Feld zu detektieren.

4. Verfahren nach einem der vorstehenden Ansprüche, **dadurch gekennzeichnet, dass** zum Bestimmen des wenigstens einen Parameters des zweiten Körperteils (26), Daten der orthopädietechnischen Einrichtung und/oder des Trägers verwendet werden.

5. Orthopädietechnische Einrichtung zum Anordnen an einer oberen Extremität (4), wobei die orthopädietechnische Einrichtung wenigstens einen Sensor (34) und eine elektrische Steuerung aufweist, die eingerichtet ist zum Durchführen eines Verfahrens nach einem der vorstehenden Ansprüche.

## Claims

1. A method for operating an orthopedic device which
- supports a first body part (24) of a wearer, and
- comprises at least one controllable actuator,
wherein the method comprises the following steps:
a) determining a chronological profile (28) of at least one parameter, which allows for a conclusion to be drawn about a movement status (30) of the wearer, from measurement values of at least one sensor (34),
b) detecting the movement status (30) from the at least one determined chronological profile (28), and
c) controlling the at least one controllable actuator depending on the detected movement status (30),
wherein
at least also the chronological profile (28) of at least one parameter of a second body part of the wearer is used to detect the movement status (30), wherein the at least one controllable actuator comprises at least one electrode for electrostimulation that is controlled in such a way that at least one arm of the wearer is caused to swing depending on the detected movement status (30), wherein the movement status (30) lasts multiple gait cycles.

2. The method according to claim 1, **characterized in that** the at least one parameter is a relative position, relative movement and/or relative speed and/or relative acceleration and/or relative angle of the second body part (26) to the first body part (24) and/or of a first part of the second body part (26) to a second part of the second body part (26).

3. The method according to one of the preceding claims, **characterized in that** the at least one sensor (34) is configured to detect an absolute angle, a relative angle, a speed, an acceleration, a force, a pressure, a pressure wave, a moment, an electrical field and/or a magnetic field.

4. The method according to one of the preceding claims, **characterized in that** data of the orthopedic device and/or the wearer is used to determine the at least one parameter of the second body part (26).

5. An orthopedic device for arranging on an upper limb (4), the orthopedic device comprising at least one sensor (34) and one electric control unit that is configured to conduct a method according to one of the preceding claims.

## Revendications

1. Procédé de fonctionnement d'un dispositif orthopédique qui
- soutient une première partie (24) du corps d'un porteur et
- comporte au moins un actionneur contrôlable,
le procédé comprenant les étapes suivantes consistant à :
a) déterminer un profil temporel (28) d'au moins un paramètre, qui permet de donner une indication sur l'état de mouvement (30) du porteur, à partir des valeurs mesurées par au moins un capteur (34),
b) reconnaître l'état de mouvement (30) du porteur à partir dudit au moins un profil temporel (28) déterminé, et
c) commander ledit au moins un actionneur contrôlable en fonction de l'état de mouvement reconnu (30) du porteur,
dans lequel
pour reconnaître l'état de mouvement (30) du porteur, on utilise au moins également le profil temporel (28) d'au moins un paramètre d'une deuxième partie (26) du corps du porteur, ledit au moins un actionneur contrôlable comprenant au moins une électrode de stimulation électrique qui est commandée de telle sorte qu'au moins un bras du porteur est amené à se balancer en fonction de l'état de mouvement reconnu (30) du porteur, sachant que l'état de mouvement (30) du porteur dure plusieurs cycles de pas.

2. Procédé selon la revendication 1,
**caractérisé en ce que** ledit au moins un paramètre est une position relative, un mouvement relatif et/ou une vitesse relative et/ou une accélération relative et/ou un angle relatif de la deuxième partie (26) du corps par rapport à la première partie (24) du corps et/ou d'une première portion de la deuxième partie (26) du corps par rapport à une deuxième portion de la deuxième partie (26) du corps.

3. Procédé selon l'une des revendications précédentes,
**caractérisé en ce que** ledit au moins un capteur (34) est conçu pour détecter un angle absolu, un angle relatif, une vitesse, une accélération, une force, une pression, une onde de pression, un couple, un champ électrique et/ou un champ magnétique.

4. Procédé selon l'une des revendications précédentes,
**caractérisé en ce que** pour déterminer ledit au moins un paramètre de la deuxième partie (26) du corps, on utilise les données du dispositif orthopédique et/ou du porteur.

5. Dispositif orthopédique destiné à être placé sur un membre supérieur (4), le dispositif orthopédique comprenant au moins un capteur (34) et une commande électrique conçue pour mettre en œuvre un procédé selon l'une des revendications précédentes.
